Europäisches Patentamt

European Patent Office

Office européen des brevets

(19)

(11) **EP 1 736 110 A1**

(12) **DEMANDE DE BREVET EUROPEEN**

(43) Date de publication:
**27.12.2006 Bulletin 2006/52**

(51) Int Cl.:
*A61B 18/20* (2006.01)   *A61B 18/22* (2006.01)

(21) Numéro de dépôt: **05370015.9**

(22) Date de dépôt: **23.06.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(71) Demandeur: **Optical System & Research for Industry and Science Osyris 59260 Hellemmes (FR)**

(72) Inventeur: **Zemmouri, Jaouad 59510 Hem (FR)**

(74) Mandataire: **Matkowska, Franck et al Matkowska & Associés 9 rue Jacques Prévert 59650 Villeneuve d'Ascq (FR)**

(54) **Moyen de calcul d'un paramètre de fonctionnement (énergie, puissance ou durée d'émission) d'un laser endoveineux**

(57)     Le moyen de calcul d'un paramètre de fonctionnement d'un laser endoveineux fournit la valeur de l'énergie (E) du faisceau laser, ou la valeur de la puissance ($P_{laser}$) du faisceau laser pour une durée d'émission prédéterminée ($t_{laser}$), ou la valeur de la durée d'émission ($t_{laser}$) du faisceau laser pour une puissance ($P_{laser}$) prédéterminée du faisceau laser, à partir de la section transversale de la veine à traiter. De préférence, la valeur du paramètre (énergie, ou puissance ou durée d'émission) est fonction d'une dimension de la section transversale de la veine à traiter, et plus particulièrement du diamètre (ou rayon) interne de la veine à traiter.

$$E(J) = 6{,}0e^{0{,}5 D_{veine}\,(mm)}$$

FIG.1

EP 1 736 110 A1

## Description

### Domaine technique

La présente invention concerne le domaine des lasers endoveineux et de leur utilisation pour le traitement local d'une veine contenant du sang, et en particulier pour collapser ou boucher localement la veine. Elle trouve principalement, mais non exclusivement, son application dans le domaine du traitement des varices.

### Art antérieur

Pour traiter des veines, et notamment des veines variqueuses, en bouchant ou en collapsant localement la veine, il est à ce jour connu d'utiliser des lasers endoveineux qui sont conçus pour être introduits à l'intérieur d'une veine. En pratique, on introduit le laser dans la veine, par exemple au moyen d'un cathéter et d'une fibre optique, jusqu'à la partie de la zone à traiter, puis une fois le laser positionné à l'intérieur de la veine, on effectue les tirs (soit en continu soit de manière discontinue) en retirant progressivement le laser sur une distance fonction de la longueur de la zone veineuse à traiter. Les longueurs d'onde usuellement utilisées sont généralement comprises dans la bande : 800nm-1000nm.

L'utilisation d'un laser endoveineux est préférable à un traitement par laser non invasif en surface, car elle permet un traitement plus efficace avec des risques plus faibles de dommages collatéraux pour les autres tissus, et permet de détruire des veines localisées plus profondément.

Dans les brevets américains US 4 564 011 et US 5 531 739, le laser endoveineux est utilisé pour coaguler localement le sang à l'intérieur de la veine, et boucher partiellement ou totalement la veine de manière localisée. Il est de ce fait primordial dans ces méthodes que la veine ne soit pas vidée de son sang.

Dans le brevet américain US 6 398 777, il est enseigné d'utiliser un laser endoveineux pour détruire localement les cellules endothéliales et la paroi interne de la veine, en sorte d'obtenir ultérieurement une fibrose locale de la veine, ce qui permet de réduire le diamètre interne de la veine, et le cas échant de collapser totalement la veine.

Selon l'enseignement de ce brevet US 6 398 777, pour obtenir l'effet recherché de fibrose de la veine, il est essentiel, préalablement au traitement laser, de vider le sang de la veine à traiter et d'amener le laser en contact avec la paroi intérieure de la veine à traiter. D'une part, cette méthode oblige à une opération supplémentaire de vidange de la veine, et l'opération de positionnement du laser au contact de la paroi interne de la veine est plus délicate. D'autre part et surtout, on constate en pratique que ce mode opératoire avec vidange de la veine ne peut être adopté, car les risques de destruction irréversible des tissus sains cutanés adjacents à la zone de la veine qui est traitée sont trop importants. Ainsi, à l'inverse de ce qui est préconisé dans ce brevet US 6 398 777, il s'avère en pratique primordial lors du traitement d'une veine par laser endoveineux, que la veine traitée contienne encore du sang.

Quelle que soit la méthode utilisée, lors d'un traitement par laser endoveineux, une difficulté importante pour le praticien réside dans le choix de l'énergie du laser qu'il convient de mettre en oeuvre. Une énergie trop faible se traduit par un traitement inefficace. Une énergie trop élevée peut aboutir de manière très préjudiciable à une destruction irréversible des tissus sains adjacents à la veine.

Aujourd'hui le réglage de l'énergie du faisceau laser (réglage de la puissance et/ou de la durée d'émission du laser) est effectué de manière empirique par le praticien en fonction de sa propre expérience. Il y a donc à ce jour un besoin non satisfait de pouvoir déterminer facilement et sans risque d'erreur l'énergie d'un laser endoveineux pour le traitement local d'une veine contenant du sang, en vue de la boucher ou de la collapser partiellement ou totalement.

### Objectif de l'invention

Un objectif de l'invention est de proposer une solution simple et efficace qui facilite le réglage d'un laser endoveineux utilisé pour le traitement d'une veine contenant du sang, en vue de boucher ou collapser partiellement ou totalement la veine.

### Résumé de l'invention

Les inventeurs ont mis en évidence que de manière surprenante il existait une relation simple entre l'énergie laser requise pour le traitement endoveineux d'une veine contenant du sang et la section transversale (notamment diamètre ou rayon) de ladite veine avant traitement.

Sur la base de ce constat, l'invention a pour premier objet un moyen de calcul d'un paramètre de fonctionnement d'un laser endoveineux.

De manière caractéristique selon l'invention, le moyen de calcul fournit la valeur de l'énergie du faisceau laser, ou la valeur de la puissance du faisceau laser pour une durée d'émission prédéterminée, ou la valeur de la durée d'émission du faisceau laser pour une puissance prédéterminée du faisceau laser, à partir de la section transversale de la veine à traiter.

Plus particulièrement, le moyen de calcul de l'invention comporte les caractéristiques additionnelles et facultatives ci-après, prises isolément ou le cas échéant en combinaison :

- la valeur du paramètre (énergie, ou puissance ou durée d'émission) est fonction d'une dimension de la section transversale de la veine à traiter ;
- la valeur du paramètre (énergie, ou puissance ou durée d'émission) est fonction du diamètre (ou rayon) interne de la veine à traiter ;
- la valeur du paramètre (énergie, ou puissance ou durée d'émission) est fonction d'une exponentielle du diamètre (ou rayon) interne de la veine à traiter ;
- le moyen de calcul est conçu pour déterminer l'énergie du faisceau laser au moyen de l'équation suivante : $E = k1 e^{k2.Dveine}$, ou pour déterminer la puissance ($P_{laser}$) du faisceau laser au moyen de l'équation suivante : $P_{laser} = (k1/t_{laser}) e^{k2.DVeine}$, ou pour déterminer la durée d'émission ($t_{laser}$) du faisceau laser au moyen de l'équation suivante : $t_{laser} = (k1/P_{laser}) e^{k2.DVeine}$, avec Dveine représentant le diamètre interne de la veine, k1 et k2 étant des constantes prédéterminées ; les valeurs de k1 et k2 dépendent notamment de la longueur d'onde du laser ;
- de préférence, k1 est compris entre 5 et 8 ;
- de préférence k2 est compris entre 0,4 et 0,6 ;
- le moyen de calcul est implémenté sous la forme d'un programme informatique enregistré sur un support mémoire ou dans une mémoire électronique ;
- le moyen de calcul est constitué par un abaque.

L'invention a également pour objet un laser endoveineux qui comporte des moyens de calcul automatique de la valeur de l'énergie (E) du faisceau laser, ou de la valeur de la puissance ($P_{laser}$) du faisceau laser pour une durée d'émission prédéterminée, ou de la valeur de la durée d'émission ($t_{laser}$) du faisceau laser pour une puissance prédéterminée du faisceau laser, lesquels moyens de calcul présentent les caractéristiques susvisées.

L'invention a pour autre objet un procédé de réglage d'un laser endoveineux.

Selon ce procédé, on règle la valeur de l'énergie (E) du faisceau laser, ou la valeur de la puissance ($P_{laser}$) du faisceau laser pour une durée d'émission prédéterminée, ou la valeur de la durée d'émission ($t_{laser}$) du faisceau laser pour une puissance prédéterminée du faisceau laser, à partir d'une mesure de la section transversale de la veine à traiter.

De préférence, pour déterminer la valeur de l'énergie (E) du faisceau laser, ou la valeur de la puissance ($P_{laser}$) du faisceau laser pour une durée d'émission prédéterminée, ou la valeur de la durée d'émission ($t_{laser}$) du faisceau laser pour une puissance prédéterminée du faisceau laser, on utilise un moyen de calcul de l'invention.

L'invention a également pour objet un procédé de traitement d'une veine au moyen d'un faisceau laser qui est introduit à l'intérieur de la veine contenant du sang jusqu'au site à traiter.

Selon ce procédé, on mesure préalablement la section transversale de la veine au niveau du site à traiter et on règle le laser conforment au procédé de réglage susvisé.

## Brève description des figures

D'autres caractéristiques et avantages de l'invention apparaîtront plus clairement à la lecture de la description détaillée ci-après, donnée à titre d'exemple non limitatif et non exhaustif de l'invention, et en référence aux dessins annexés sur lesquels :

- la figure 1 est un exemple d'abaque de l'invention fournissant l'énergie laser en fonction du diamètre interne ($D_{veine}$) de la veine,
- la figure 2 représente de manière schématique une veine et la fibre laser introduite dans la veine,
- la figure 3 est une coupe transversale de la veine de la figure 2,
- les figures 4 à 10 sont des courbes d'isodommage obtenues pour différents rayons internes de veine,
- La figure 11 est un synoptique d'un laser équipé de moyens de réglage automatique spécifiques de l'invention.

## Description détaillée

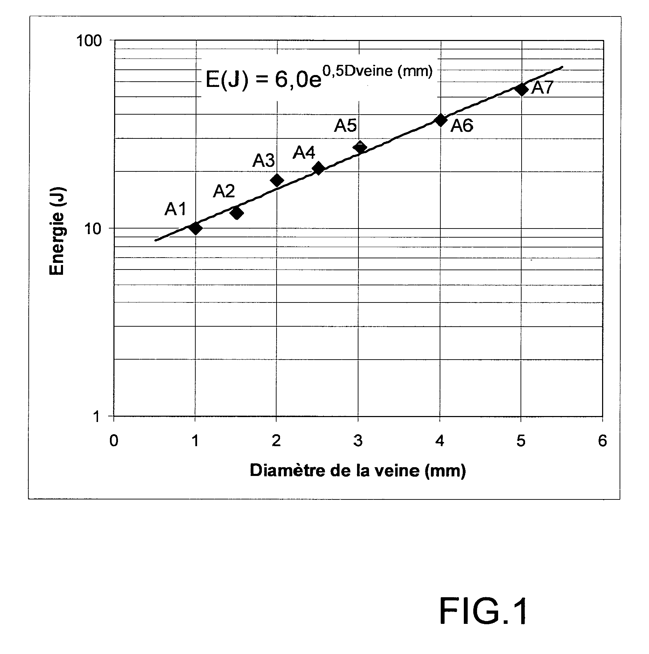

La figure 1 est un abaque fournissant, dans le domaine du traitement par laser d'une veine par voie endoveineuse, un exemple de relation simple entre l'énergie E requise pour le faisceau laser et la section transversale de la veine à traiter, et plus particulièrement dans cet exemple le diamètre interne Dveine de la veine à traiter. Cette relation de la figure 1 a été obtenue par calcul numérique sur la base du modèle géométrique ci-après et au moyen de la méthode de calcul numérique détaillée ci-après.

Modèle géométrique

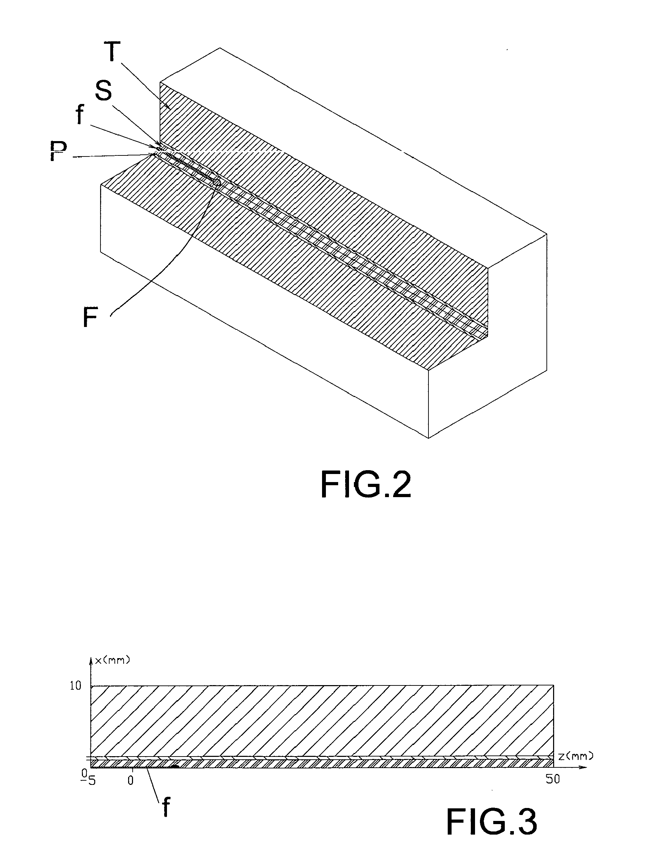

Pour les calculs, on utilise le modèle géométrique des figures 2 et 3 . Dans ce modèle, la veine à traiter est un cylindre comportant une paroi (P), contenant du sang (S) et entourée de tissu (T). Sur la figure 2, la fibre du laser est représentée de manière schématique et est référencée (f).

Etant donné que le système présente une symétrie de révolution centrée sur l'axe longitudinal de la veine, les calculs sont effectués dans une section à deux dimensions ayant les paramètres de la figure 3. [Epaisseur de la paroi de la veine : $E_{veine}$; Rayon interne de la veine: Rveine ].

Sur la figure 2, la fibre optique du laser est représentée de manière schématique et est référencée (f). On considère que le faisceau laser F en sortie de cette fibre optique f est centré sur l'axe central longitudinal de la veine. On considère en outre que la longueur d'onde du faisceau laser F est comprise 800nm et 1000nm, et vaut par exemple 980nm.

Pour calculer la répartition du faisceau laser dans l'espace, on considère que la propagation du faisceau laser est principalement diffusée par le milieu (régime diffusant dominant), conformément à l'enseignement de la publication suivante :

M.N. Iizuka, I.A. Vitkin, M.C. Kolios, M.D. Sherar, « The effects of dynamic optical properties during interstitial laser photocoagulation', Phys. Med. Biol. 45 (2000) 1335-1357.

La répartition de la puissance émise en un point donné est donnée par l'équation suivante :

$$(1) \quad \phi(r) = \frac{P_{Laser}.\exp(-\mu_{eff}.r)}{4\pi.D.r}$$

Où:

$P_{Laser}$ est la puissance en Watts du faisceau laser ;
$\mu_{eff}$ est le coefficient d'absorption effectif (en mm$^{-1}$) ;
r est la distance (en mm) par rapport au point d'émission du faisceau laser (c'est-à-dire en pratique point de sortie de la fibre du laser) ;
D caractérise la diffusion (en mm).

$\mu_{eff}$ est défini par l'équation suivante :

$$(2) \quad \mu_{eff} = \sqrt{3.\mu_a(\mu_a + \mu'_s)}$$

où :

$\mu_a$ est le coefficient d'absorption (en mm$^{-1}$) ;
$\mu'_s$ est le coefficient de diffusion réduit défini lui-même par l'équation suivante :

$$(3) \quad \mu'_s = \mu_s.(1-g) ,$$

où :

$\mu_s$ est le coefficient de diffusion (en mm$^{-1}$);
$g$ est le facteur de diffusion anisotrope (sans unité).

Les valeurs de $\mu_a$ et $\mu'_s$ varient en fonction de la longueur d'onde du faisceau laser.
D est défini par la relation suivante :

$$(5)\ D = \frac{1}{3(\mu_a + \mu'_s)} = \frac{\mu_a}{\mu_{eff}^2}$$

r est défini par la relation suivante :

$$(6)\ r = \sqrt{x^2 + z^2}$$

où :

x (en mm) est la distance radial mesurée selon l'axe X (figure 3) par rapport au point d'émission du faisceau laser ;
z (en mm) est la distance longitudinale mesurée selon l'axe Z (figure 3) par rapport au point d'émission du faisceau laser.

La puissance absorbée (en W /mm$^3$) en chaque point est calculée en utilisant la relation suivante :

$$(7)\ P_{abs} = \mu_a . \phi(r)$$

Lors des calculs, on considère que le premier tir laser se fait toujours au point de coordonnées (0,0) de la figure 3. Lorsque le mode « multi pulse » est simulé, on introduit une variable z' qui représente la position relative dans la veine et qui est défini par la relation suivante :

$$(8)\ z' = z - z_{inc}$$

où : $z_{inc}$ (en mm) représente la position absolue.
Pour calculer la position absolue $z_{inc}$ de chaque tir selon l'axe longitudinal Z, on utilise un compteur qui est multiplié par la distance entre chaque tir (distance de retrait de la fibre (f) du laser).
Dans le cas d'un tir dit continu avec retrait simultané et progressif de la fibre (f) du laser, on remplace dans la formule (8) précitée la variable $Z_{inc}$ par la formule :

$$(9)\ Z_{inc} = t.v,$$

Où:

- v représente la vitesse de retrait de la fibre ;
- t représente le temps.

Paramètres optiques

Seule l'énergie du laser [puissance du laser ($P_{laser}$) et/ou durée d'émission du laser] est une variable ; les valeurs des autres paramètres sont fixées conformément au tableau I ci-dessous et sont définis pour une longueur d'onde de 980nm :

Tableau I :

| Milieu | $\mu_a$ (mm$^{-1}$) | $\mu'_s$ (mm$^{-1}$) | $\mu_{eff}$(mm$^{-1}$) |
|---|---|---|---|
| **Sang (S)** | 0,25 | 0,60 | 0,80 |
| **Paroi (P)** | 0,1 | 2,0 | 0,80 |
| **Tissu (T)** | 0,030 | 1,0 | 0,30 |

Paramètres thermiques

Pour le calcul de l'élévation de température et de la diffusion thermique, on utilise l'équation de la chaleur ci-après :

$$(10) \quad \nabla.(k.\nabla T(r,t)) + P_{abs} = C_p.\frac{\partial T(r,t)}{\partial t}$$

où:

$T(r,t)$ représente la température en un point donné et à un instant donné ;
$k$ (en W.mm$^{-1}$.K$^{-1}$) représente la conductivité thermique ;
$C_p$ (J .mm$^{-3}$.K$^{-1}$) représente la capacité thermique volumique définie par la relation suivante :

$$(11) \quad C_p = C.\rho$$

Où:

$C$ (J.g$^{-1}$.K$^{-1}$) est la capacité thermique massique,
$\rho$ (g.mm$^{-3}$) est la densité du milieu.

Ces paramètres sont fixés avec les valeurs du tableau II ci-après, la température ambiante étant en outre fixée à 37°C :

Tableau II

| Milieu | $C$ (J.g$^{-1}$.K$^{-1}$) | $\rho$ (g.mm$^{-3}$) | $k$ (en W.mm$^{-1}$.K$^{-1}$) |
|---|---|---|---|
| **Sang (S)** | 3,82 | 1,05.10$^{-3}$ | 5,6.10$^{-4}$ |
| **Paroi (P)** | 3,78 | 1,05.10$^{-3}$ | 5,6.10$^{-4}$ |
| **Tissu (T)** | 3,78 | 1,05.10$^{-3}$ | 5,6.10$^{-4}$ |

Transition de phase du sang

Etant donné que le sang est constitué comme la plupart des autres tissus, d'une part importante d'eau, au-dessus de 100°C le sang possède une transition de phase. Etant donné que la veine est un milieu fermé mais déformable, il est difficile que la température du sang dépasse 100°C. Par conséquent, une limitation a été introduite dans le modèle consistant à augmenter fortement la capacité thermique lorsque la température du milieu dépasse 100°C.

Calcul des courbes d'isodommage

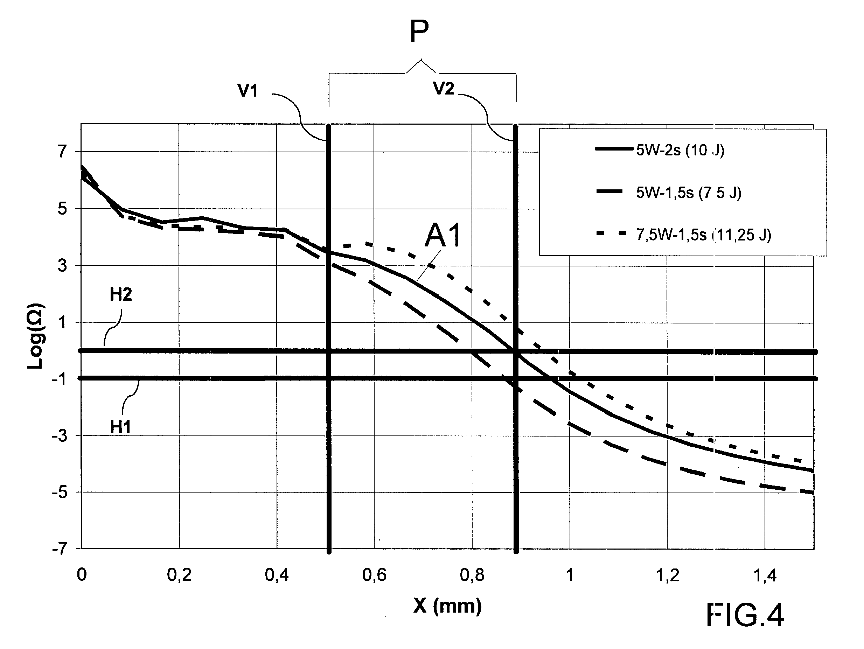

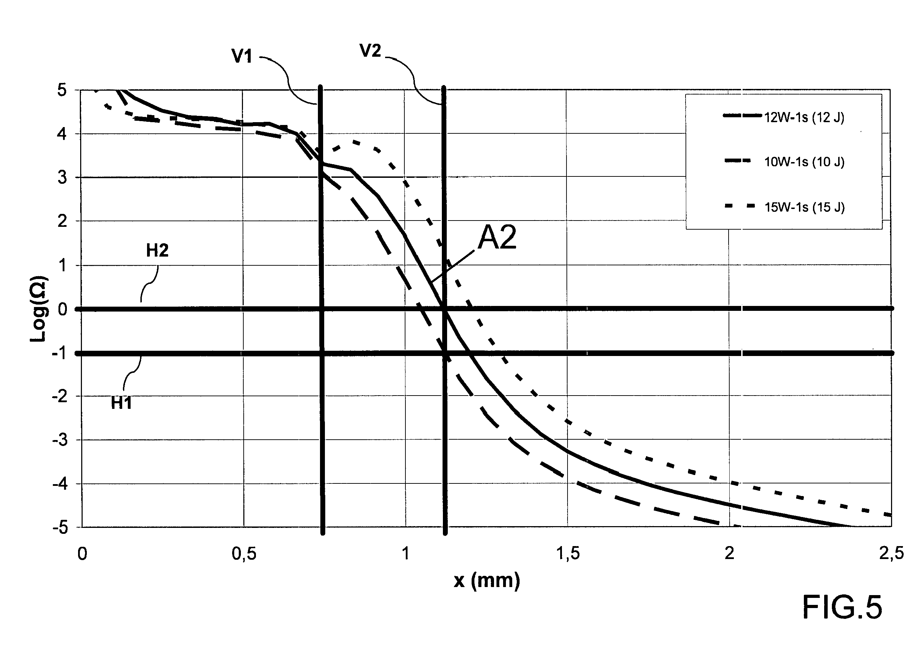

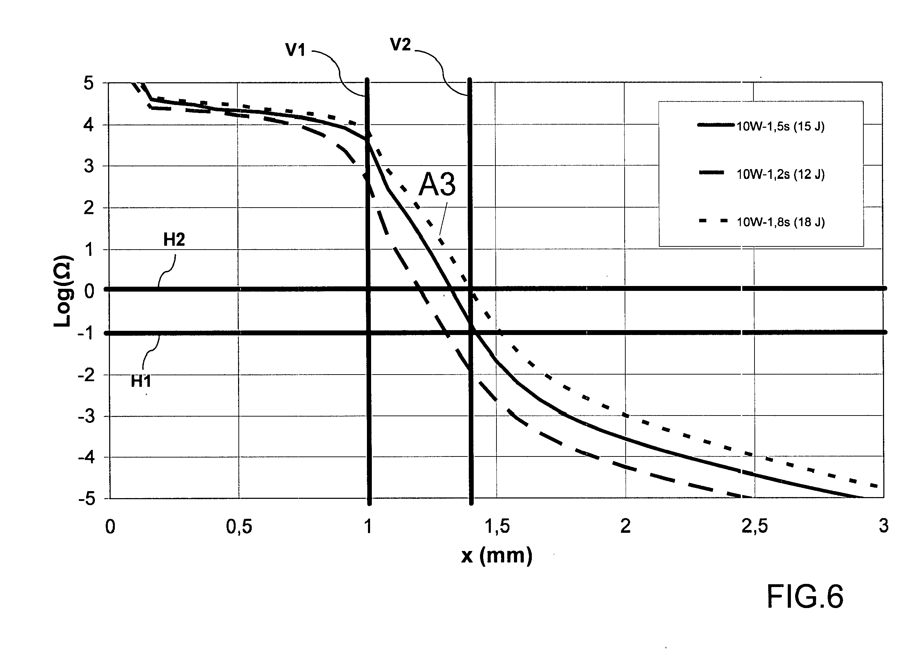

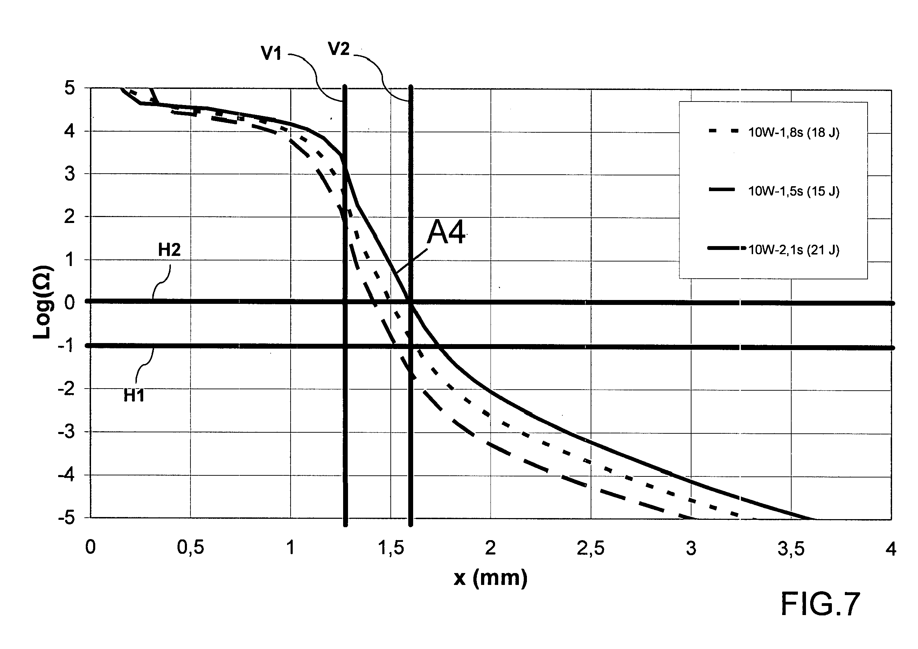

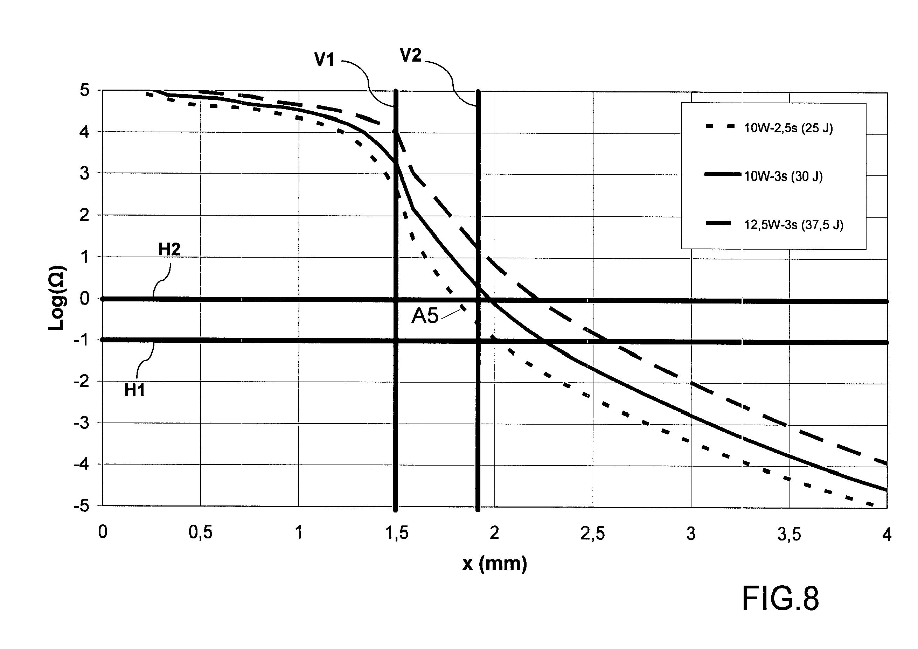

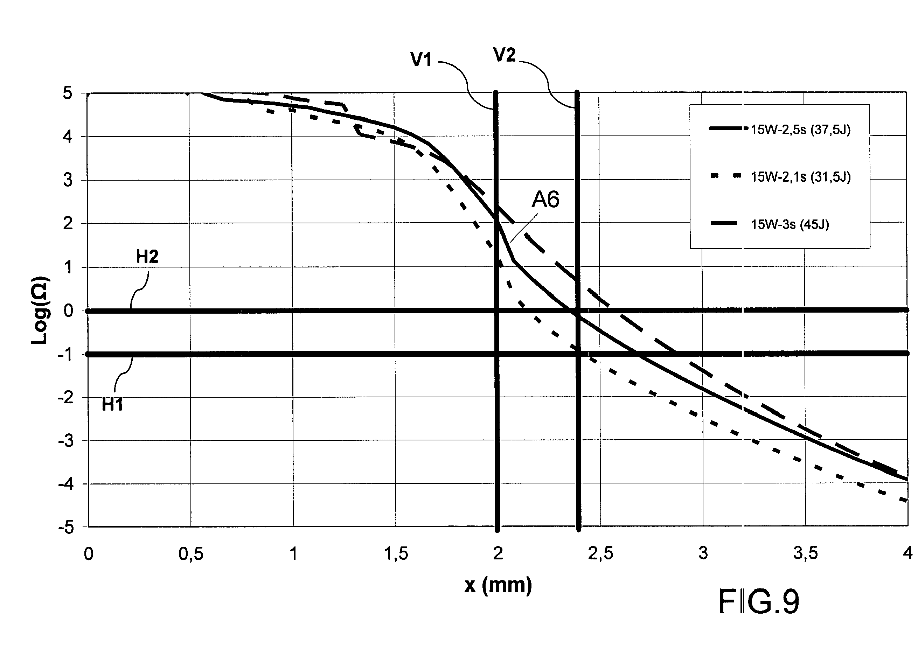

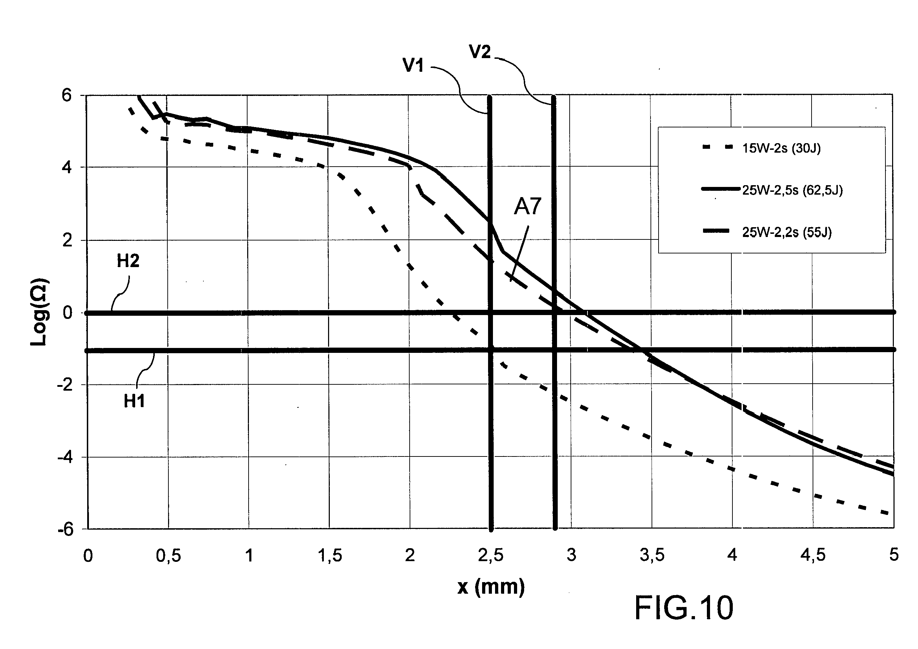

On détermine par calcul numérique les courbes dites d'isodommage des figures 4 à 10, pour différents rayons internes de veine. Les figures 4 à 10 représentent les courbes d'isodommage pour des veines présentant respectivement un rayon interne valant : 0,5mm ; 0,75mm; 1mm; 1,25mm; 1,5mm; 2mm; 2,5mm. Chaque courbe d'isodommage des figures 4 à 10 est calculée pour un couple déterminé (puissance du faisceau laser en Watts - durée d'émission du faisceau laser en secondes).

Pour calculer ces courbes d'isodommage des figures 4 à 10, on utilise la relation d'Arrhenius ci-après, selon laquelle l'effet de la température sur un tissu dépend uniquement de deux paramètres : la valeur de la température et le temps pendant lequel cette température est maintenue.

$$(12) \quad \log(\Omega) = \log(A) + \log[\int_0^t \exp(\frac{-Ea}{R.T(r,t)})dt]$$

Le paramètre $\Omega$ peut également être exprimé par la relation suivante :

$$(13) \quad \Omega = -\ln\frac{C(t)}{C_0}$$

où :

$C_0$ représente la concentration initiale des cellules du tissu,
$C(t)$ représente la concentration des cellules non endommagées à un instant t.

Le critère habituellement utilisé pour déterminer la zone maximale de dégât est de considérer que lorsque les deux tiers (soit environ 66%) du volume sont endommagés, l'effet est irréversible. Dans ce cas le paramètre $\Omega$ est égal à 1 (voir infra/ droite H2 des les figures 4 à 10).

On introduit également un second critère en considérant que lorsque 10% du volume sont endommagés, cela n'est pas suffisant pour produire un effet irréversible, mais cela provoque un échauffement qui peut être ressenti par le patient ( voir infra / droite H1 des les figures 4 à 10).

Les paramètres $E_a$ (énergie d'activation) et A (susceptibilité thermique) sont fixés avec les valeurs du tableau III ci-après.

Tableau III

| Milieu | $E_a$ (J.mol$^{-1}$) | A(s$^{-1}$) |
|---|---|---|
| **Sang (S)** | $4,48.10^5$ | $7,6.10^{66}$ |
| **Paroi (P)** | $4,30.10^5$ | $5,6.10^{66}$ |
| **Tissu (T)** | $4,30.10^5$ | $5,6.10^{66}$ |

Pour les calculs, on a considéré également que l'on effectuait un tire laser dans la veine toutes les 5 secondes (intervalle de temps entre deux tirs successifs), et qu'entre chaque tire on déplaçait le faisceau laser de 3mm (distance de retrait longitudinale en Z de la fibre (f) du laser entre chaque tir).

Sur les figures 4 à 10, on a représenté par deux traits verticaux V1 et V2 respectivement la position des deux faces interne et externe de la paroi P de la veine. A gauche du trait vertical V1, on se situe dans le sang (S). A droite du trait vertical V2, on se situe dans le tissu (T).

Sur les figures 4 à 10, on a représenté par deux trait horizontaux H1 et H2, les deux seuils précités de dégâts : le trait vertical H1 correspond à 10% de tissus endommagés ; le trait vertical H2 correspond à 66% de tissus endommagés.

On considère que dans la zone située entre les deux traits horizontaux, les tissus sont chauffés et donc en partie dénaturés mais pas de manière irréversible. Au-delà de la limite de 66% (trait horizontal H2), on considère de manière connue en soi que la destruction des tissus est irréversible.

Les points A1 à A7 de la figure 1 sont obtenus à partir des courbes isodommage des figures 4 à 10 de la manière suivante. On retient pour chaque figure la courbe d'isodommage qui coupe la limite des 66% de dommage (trait horizontal H2) à gauche du trait vertical V2 (face externe de la paroi veineuse) et qui se situe au plus près de la face externe V2 de la paroi veineuse P.

Par interpolation, l'équation de la courbe reliant les points A1 à A7 de la figure 1 est:

$$(14) \quad E = k_1 e^{k_2 . Dveine}$$

où :

E est l'énergie du faisceau laser exprimée en Joules;
$D_{veine}$ est le diamètre interne en mm de la veine ;
k1 est égal 6 et k2 est égal à 0,5. Ces valeurs de k1 et k2 correspondent à une longueur d'onde de 980nm.

Bien entendu, cette équation mathématique particulière obtenue par interpolation linéaire, et notamment les valeurs précises ci-dessus pour les coefficients k1 et k2, ne sont pas limitatives de l'invention. En suivant la même démarche de simulation, mais en retenant pour établir la figure 1, d'autres courbes d'isodommage intersectant la limite des 66% de dommage (trait horizontal H2) dans la région de la paroi veineuse (P) (entre les deux traits verticaux V1 et V2), on peut le cas échéant obtenir une équation qui est différente, mais qui permet néanmoins d'exprimer simplement la relation entre l'énergie requise pour le faisceau laser ( dans l'exemple particulier décrit pour obtenir sensiblement 66% de destruction des tissus de la paroi veineuse P) en fonction du diamètre interne de la veine. Egalement, on peut utiliser la même méthode, mais en optant pour une limite de dégât autre que 66%, la limite de 66% n'étant qu'une valeur préférentielle permettant d'obtenir des effets irréversibles sur la paroi veineuse P.

Des simulations ont également été effectuées en faisant varier la valeur du coefficient d'absorption $\mu_a$ du sang, ce qui revient indirectement à modifier la longueur d'onde du faisceau laser. Les résultats obtenus pour les coefficients k1 et k2 de l'équation (14) sont donnés dans le tableau IV ci-après.

**Tableau IV**

| $\mu_a$(sang) | k1 | k2 |
|---|---|---|
| 0,1 | 5,967 | 0,5765 |
| 0,15 | 5,8322 | 0,5741 |
| 0,2 | 7,503 | 0,4442 |
| 0,35 | 6,2736 | 6,2736 |

On peut noter sur le graphe de la figure 1 que pour une veine présentant un diamètre interne trop important (typiquement au delà de 5mm pour le diamètre interne de la veine), l'énergie laser requise pour détruire la paroi veineuse devient trop importante ; ceci s'explique par la présence dans la veine d'une quantité de sang trop importante, qui absorbe de manière importante l'énergie laser diffusée dans le sang avant que celle-ci n'atteigne la paroi veineuse. En pratique, pour des veines présentant un diamètre interne trop important (typiquement supérieur à 5mm), l'énergie laser requise pour détruire la paroi veineuse est trop élevée, et le traitement de ce type de veine n'est donc pas envisageable avec une énergie laser acceptable.

L'invention n'est pas limitée à un calcul d'énergie du faisceau laser en fonction du diamètre interne de veine, mais d'une manière plus générale couvre tout moyen de calcul de l'énergie (de la puissance ou de la durée d'émission) d'un faisceau laser endoveineux en fonction de la section transversale de la veine (notamment diamètre ou rayon, ou surface de la section transversale)

_Méthode de traitement des varices ou similaire avec réglage du faisceau laser en fonction de la section transversale de la veine_

On peut utiliser tout type connu de laser endoveineux fonctionnant notamment (mais pas exclusivement) dans les longueurs d'onde 800nm-1000nm, quelle que soit la structure du laser et quel que soit le moyen associé (cathéter ou autre) utilisé pour faire pénétrer le faisceau laser à l'intérieur de la veine. Le laser utilisé peut être de type pulsé ou de type continu.

Généralement le laser endoveineux comporte une source laser qui permet un réglage manuel par le praticien de la durée d'émission ($t_{laser}$) du faisceau et/ou de la puissance d'émission du faisceau laser ($P_{laser}$), et une sortie fibrée pour le laser.

L'utilisation du laser se fait en deux temps:

- réglage du laser en fonction de la veine à traiter

- utilisation du laser réglé pour traiter la veine par voie endoveineuse.

Réglage du laser

Pour le réglage préalable du laser, on fournit à l'utilisateur du laser chargé des opérations de réglage un support (papier au autre) comportant au moins un abaque du type de celui de la figure 1 (spécifique en l'espèce pour un intervalle de temps de 5 secondes entre chaque tir et d'une distance de retrait de 3mm).

La méthode de réglage est mise en oeuvre selon les étapes successives suivantes :

(a) On localise la région veineuse à traiter (varice) et on mesure par exemple par échographie le diamètre interne de la veine dans cette région ;
(b) Au moyen de l'abaque (figure 1), on détermine l'énergie E requise pour le faisceau laser.
(c) On règle, la durée d'émission ($t_{laser}$) et/ou la puissance ($P_{laser}$) du laser pour obtenir l'énergie (E) requise, étant rappelé que :

$$E(J) = P_{laser}(W).t_{laser}(s)$$

Il convient de noter que ces étapes de réglage (a) à (c) ne sont pas nécessairement mises en oeuvre par le phlébologue, mais peuvent être réalisées par un technicien n'ayant pas nécessairement de compétences chirurgicales.

Traitement

Une fois le laser réglé, le traitement de la veine est réalisé de la manière suivante :

(d) On introduit la fibre optique du laser au moyen d'un cathéter dans la veine jusqu'au point d'émission initial le plus éloigné dans la région veineuse à traiter, le patient ayant préalablement subi une anesthésie locale ou générale; la sortie de la fibre optique du laser n'est pas en contact avec la paroi interne de la veine ; en outre, on s'assure de préférence que le point initial d'émission est situé à au moins à 1 cm, et plus préférentiellement à au moins 2cm de la jonction saphénofémorale.
(e) On déclenche un premier tir.
(f) On procède au retrait de la fibre optique du laser d'un pas fixé par l'abaque (3mm dans le cas de la figure 1), puis après un laps de temps déterminé ( 5 secondes dans le cas de l'abaque de la figure 1 ) on procède à un nouveau tir. Cette étape est réitérée autant de fois que nécessaire pour couvrir sur toute sa longueur la région veineuse devant être traitée.

On obtient ainsi une destruction locale et irréversible de la paroi veineuse qui se traduit par une obturation de préférence (mais pas nécessairement) complète de la veine dans la région traitée.

Dans une variante de réalisation, l'étape (d) est réalisée avant la première étape (a) de réglage, de telle sorte que la mesure du diamètre interne de la veine est avantageusement effectuée lorsque la fibre du laser et son cathéter sont déjà en place dans la veine.

Eventuellement, lorsque la pression sanguine dans la région à traiter déforme de manière trop importante la veine (diamètre interne de veine trop important), on peut préalablement au traitement incliner par rapport à l'horizontal le patient en position dite de Tredelenbourg en sorte de faire diminuer cette pression et par là-même de diminuer légèrement le diamètre interne initial de la veine préalablement au traitement.

L'abaque de la figure 1 peut être remplacé par un abaque qui fournit directement la valeur du ou des paramètres réglables de la source laser, c'est-à-dire la valeur de la puissance du faisceau laser ($P_{laser}$) pour une durée d'émission prédéterminée ($t_{laser}$), ou la valeur de la durée d'émission du faisceau laser ($t_{laser}$) pour une puissance prédéterminée ($P_{laser}$) du faisceau laser, ce qui évite de devoir calculer ces paramètres à partir de l'énergie (E).

Dans une autre variante de réalisation de l'invention, l'équation reliant l'énergie (E) au diamètre interne de la veine peut être implémentée sous la forme d'un programme informatique, qui est fourni à l'utilisateur par exemple en étant enregistré sur un support mémoire de préférence amovible du type CDROM ou disquette. Il suffit à l'utilisateur de charger ce programme en mémoire d'un micro-ordinateur usuel du commerce. Ce programme est utilisé à l'étape (b) précitée pour régler le laser. Ce programme est par exemple conçu dans une première étape pour demander à l'utilisateur via l'écran de l'ordinateur, de saisir la valeur du paramètre d'entrée (Dveine), et pour permettre à l'utilisateur, au moyen du clavier de l'ordinateur, de saisir ladite valeur. A partir de cette valeur entrée par l'utilsateur, le programme calcule au moyen de la formule mathématique précitée la valeur de l'énergie E correspondante, ou le cas échéant la valeur de la

puissance ($P_{laser}$) du faisceau laser pour une durée d'émission prédéterminée ($t_{laser}$), ou la valeur de la durée d'émission du laser ($t_{laser}$) pour une puissance prédéterminée du faisceau laser ($P_{laser}$).

Egalement dans une autre variante de réalisation, on peut concevoir un laser endoveineux qui incorpore un programme informatique conforme à l'invention pour le calcul automatique de la valeur de l'énergie du faisceau laser (E) ou de la valeur de la puissance ($P_{laser}$) du faisceau laser pour une durée d'émission ($t_{laser}$) prédéterminée, ou de la valeur de la durée d'émission ($t_{laser}$) du faisceau laser pour une puissance ($P_{laser}$) prédéterminée du faisceau laser. Ce programme est par exemple stocké dans une mémoire électronique (du type RAM, EPROM,..) du laser endoveineux laquelle mémoire est accessible en lecture par un processeur (par exemple microprocesseur ou microcontrôleur) du laser endoveineux.

Dans une réalisation simple, le réglage du paramètre de fonctionnement du laser (énergie, puissance ou durée d'émission) est effectué manuellement à partir de la valeur fournie automatiquement, par exemple sur un écran du laser endoveineux, à partir de la valeur du diamètre (Dveine) entrée dans les moyens de calcul du laser endoveineux.

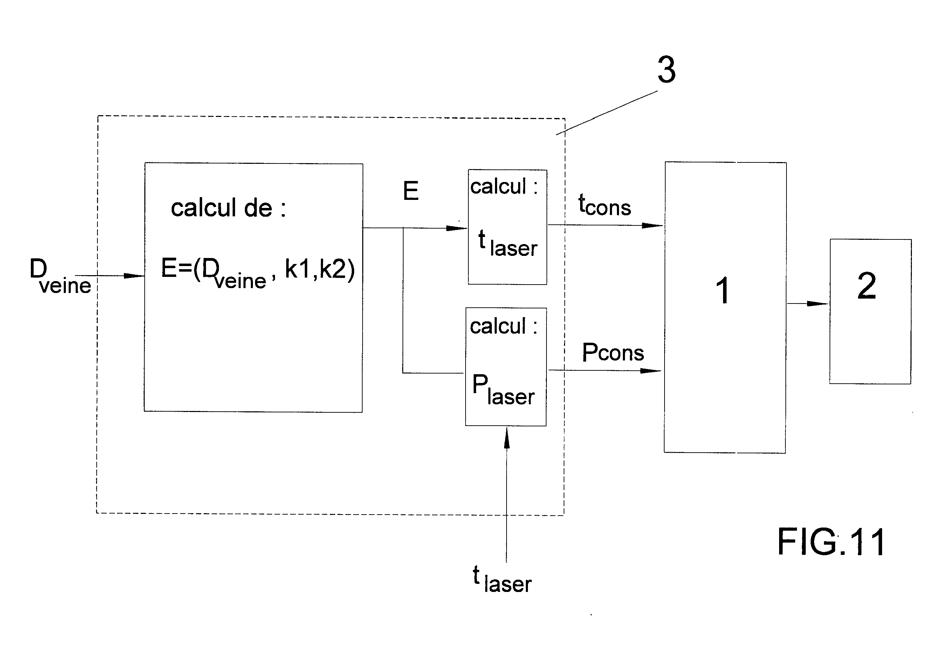

Dans une réalisation plus sophistiquée (figure 11), le laser endoveineux comporte des moyens 1 de réglage automatique du paramètre de fonctionnement du laser (par exemple durée d'émission ou puissance), lesquels moyens de réglage pilotent automatiquement la source laser 2 en en fonction d'une valeur de consigne d'entrée (t ou P) qui est délivrée par des moyens de calcul automatique 3.

## Revendications

1.  Moyen de calcul d'un paramètre de fonctionnement d'un laser endoveineux, **caractérisé en ce qu'**il fournit la valeur de l'énergie (E) du faisceau laser, ou la valeur de la puissance ($P_{laser}$) du faisceau laser pour une durée d'émission prédéterminée ($t_{laser}$), ou la valeur de la durée d'émission ($t_{laser}$) du faisceau laser pour une puissance ($P_{laser}$) prédéterminée du faisceau laser, à partir de la section transversale de la veine à traiter.

2.  Moyen de calcul selon la revendication 1 **caractérisé en ce que** la valeur du paramètre (énergie, ou puissance ou durée d'émission) est fonction d'une dimension de la section transversale de la veine à traiter.

3.  Moyen de calcul selon la revendication 2 **caractérisé en ce que** la valeur du paramètre (énergie, ou puissance ou durée d'émission) est fonction du diamètre (ou rayon) interne de la veine à traiter.

4.  Moyen de calcul selon la revendication 3 **caractérisé en ce que** la valeur du paramètre (énergie, ou puissance ou durée d'émission) est fonction d'une exponentielle du diamètre (ou rayon) interne de la veine à traiter.

5.  Moyen de calcul selon la revendication 4 **caractérisé en ce qu'**il est conçu pour déterminer l'énergie du faisceau laser au moyen de l'éqùation suivante : $E = k1e^{k2.Dveine}$, ou pour déterminer la puissance ($P_{laser}$) du faisceau laser au moyen de l'équation suivante : $P_{laser} = (k1/t_{laser})e^{k2.DVeine}$, ou pour déterminer la durée d'émission ($t_{laser}$) du faisceau laser au moyen de l'équation suivante : $t_{laser} = (k1/P_{laser})e^{k2.DVeine}$, avec Dveine représentant le diamètre interne de la veine, k1 et k2 étant des constantes prédéterminées.

6.  Moyen de calcul selon la revendication 5 **caractérisé en ce que** k1 est compris entre 5 et 8.

7.  Moyen de calcul selon la revendication 5 ou 6 **caractérisé en ce que** k2 est compris entre 0,4 et 0,6.

8.  Moyen de calcul selon l'une des revendications 1 à 7 **caractérisé en ce qu'**il est implémenté sous la forme d'un programme informatique enregistré sur un support mémoire ou dans une mémoire électronique.

9.  Moyen de calcul selon l'une des revendications 1 à 8 **caractérisé en ce qu'**il est constitué par un abaque.

10. Laser endoveineux **caractérisé en ce qu'**il comporte des moyens de calcul automatique (3) de la valeur de l'énergie (E) du faisceau laser, ou de la valeur de la puissance ($P_{laser}$) du faisceau laser pour une durée d'émission prédéterminée, ou de la valeur de la durée d'émission ($t_{laser}$) du faisceau laser pour une puissance prédéterminée du faisceau laser, lesquels moyens de calcul sont conformes à l'une des revendications 1 à 8.

11. Procédé de réglage d'un laser endoveineux **caractérisé en ce qu'**on règle la valeur de l'énergie (E) du faisceau laser, ou la valeur de la puissance ($P_{laser}$) du faisceau laser pour une durée d'émission prédéterminée, ou la valeur de la durée d'émission ($t_{laser}$) du faisceau laser pour une puissance prédéterminée du faisceau laser, à partir d'une mesure de la section transversale de la veine à traiter.

**12.** Procédé selon la revendication 11 **caractérisé en ce que** pour déterminer la valeur de l'énergie (E) du faisceau laser, ou la valeur de la puissance ($P_{laser}$) du faisceau laser pour une durée d'émission prédéterminée, ou la valeur de la durée d'émission ($t_{laser}$) du faisceau laser pour une puissance prédéterminée du faisceau laser, on utilise un moyen de calcul visé à l'une quelconque des revendications 1 à 9.

FIG.1

T
S
f
P

F

**FIG.2**

x(mm)

10

z(mm)

0
−5    0                                      50

f

**FIG.3**

FIG.4

FIG.5

EP 1 736 110 A1

FIG.6

Legend:
- 10W-1,5s (15 J)
- 10W-1,2s (12 J)
- 10W-1,8s (18 J)

x (mm)

Log(Ω)

V2, V1, A3, H2, H1

FIG.7

FIG.8

EP 1 736 110 A1

FIG.9

Legend:
- 15W-2,5s (37,5J)
- 15W-2,1s (31,5J)
- 15W-3s (45J)

FIG.10

FIG.11

**Office européen**

**des brevets**

## RAPPORT DE RECHERCHE EUROPEENNE

Numéro de la demande

EP 05 37 0015

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2004/199151 A1 (NEUBERGER WOLFGANG [MY]) 7 octobre 2004 (2004-10-07) * alinéas [0004], [0022], [0029], [0034], [0037], [0038], [0041], [0043], [0046], [0050] - [0052]; revendication 17; figure 1 * | 1-3,8-12 | INV. A61B18/20 A61B18/22 |
| X | US 6 398 777 B1 (NAVARRO LUIS ET AL) 4 juin 2002 (2002-06-04) * alinéas [0133], [0157] - [0160], [0278], [0332] * | 1-3,8-12 | |
| D,A | IIZUKA M N ET AL: "The effects of dynamic optical properties during interstitial laser photocoagulation" PHYSICS IN MEDICINE AND BIOLOGY IOP PUBLISHING UK, vol. 45, no. 5, mai 2000 (2000-05), pages 1335-1357, XP002378679 ISSN: 0031-9155 Extrait de l'Internet: URL:http://ej.iop.org/links/q21/h82h5D5la3 LkcOb813vU9g/m00517.pdf> Section 2. | 4 | DOMAINES TECHNIQUES RECHERCHES (IPC) A61B A61N |

Le présent rapport a été établi pour toutes les revendications

| Lieu de la recherche | Date d'achèvement de la recherche | Examinateur |
|---|---|---|
| Berlin | 27 avril 2006 | Kronberger, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.......................................................................

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 05 37 0015

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-04-2006

| Document brevet cité au rapport de recherche | Date de publication | Membre(s) de la famille de brevet(s) | Date de publication |
|---|---|---|---|
| US 2004199151 A1 | 07-10-2004 | EP 1613393 A2<br>WO 2004089462 A2 | 11-01-2006<br>21-10-2004 |
| US 6398777 B1 | 04-06-2002 | AUCUN | |

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4564011 A
- US 5531739 A

- US 6398777 B

**Littérature non-brevet citée dans la description**

- **M.N. LIZUKA ; I.A. VITKIN ; M.C. KOLIOS ; M.D. SHERAR.** The effects of dynamic optical properties during interstitial laser photocoagulation. *Phys. Med. Biol.,* 2000, vol. 45, 1335-1357